# FASCICULE DE BREVET EUROPEEN

(11) **EP 0 746 325 B1**
(45) Date de publication et mention de la délivrance du brevet: **25.09.2002**
(21) Numéro de dépôt: 95910594.1
(22) Date de dépôt: 24.02.1995
(51) Int. Cl.: A61K 31/70, A61K 9/107, A61K 47/06

(54) **GELEE LAXATIVE HYPOCALORIQUE A BASE D'HUILE DE PARAFFINE LIQUIDE ET DE LACTULOSE, SON PROCEDE DE PREPARATION**
GELIERTES HYPERKALORISCHES LAXIERENDMITTEL ENTHALTEND FLÜSSIGES PARAFFINÖL UND LACTULOSE, UND VERFAHREN
LIQUID PARAFFIN OIL AND LACTULOSE-BASED HYPOCALORIC LAXATIVE JELLY AND METHOD FOR ITS PREPARATION

(30) Priorité: 25.02.1994 FR 9402132
(43) Date de publication de la demande: 11.12.1996
(73) Titulaire: Pfizer, 75014 Paris (FR)
(72) Inventeur: DOAT, Bernard, F-49000 Angers (FR)
(74) Mandataire: Dufresne, Guillaume Alain François
(86) Numéro de dépôt international: FR9500219
(87) Numéro de publication internationale: WO95022976

(56) Documents cités:
- EP-A- 0 216 557
- EP-A- 0 478 837
- EP-A- 0 486 353
- FR-A- 2 618 351
- DATABASE WPI Week 9330, Derwent Publications Ltd., London, GB; AN 93-239922 (30) & JP,A,05 163 151 (TEIKOKU SEIYAKU KK) 29 Juin 1993

## Description

### Domaine de l'invention.

La présente invention a pour objet une nouvelle composition émulsifiée à base d'huile de paraffine liquide et de lactulose, obtenue sous la forme d'une gelée souple, non collante et destinée à être administrée par voie orale pour le traitement de la constipation chez l'homme.

### Arrière plan technologique de l'invention.

La constipation est un phénomène pathologique courant qui affecte un nombre considérable d'individus sans distinction réelle de sexe ou d'âge. Des publications récentes font état que ce phénomène affecte de façon permanente au moins 10% des populations françaises et britanniques (CHAUSSADE S. et GUERRE J. - Traitement de la constipation. - Encycl.Méd.Chir. Paris, France), Estomac-Intestin, 9071 A¹⁰, 6-1985, 6 p. ; TAYLOR R. - Br.Med.J. - 1990;**300**:1063-4) et que ce pourcentage est encore plus important aux Etats-Unis, au Japon et dans les pays du nord de l'Europe. Il est également connu que près de 50% des personnes atteintes pratiquent l'auto-médication par des laxatifs dont certains sont considérés potentiellement dangereux.

Hormis les cas les plus simples, dans lesquels une correction alimentaire, notamment par l'administration de fibres naturelles ou diététiques, suffit à normaliser la situation, il est fréquemment nécessaire d'intervenir par des moyens laxatifs dont l'un des plus anciens et des moins nocifs consiste en l'ingestion d'une huile minérale, plus particulièrement d'huile de paraffine liquide.

Il est reconnu que cette huile, non absorbée, agit par ses propriétés lubrifiantes et émollientes et ainsi aide à l'évacuation des fèces. Cette activité a été et est encore largement appliquée puisqu'au dictionnaire français des spécialités pharmaceutiques (DICTIONNAIRE VIDAL - 1993 - Editions du Vidal) il est répertorié une douzaine de spécialités contenant des huiles de paraffine de viscosités différentes, associées ou non à d'autres composés, et proposées sous formes liquides, gélifiées ou bien de poudres solides.

Les produits liquides sont en fait le plus souvent de l'huile de paraffine pure ou aromatisée. Ce sont les moins onéreux. Toutefois, outre les difficultés pratiques à leur manipulation, leur administration aux personnes alitées ou aux jeunes enfants est délicate car, en cas de déglutition perturbée, leur inhalation involontaire peut provoquer des affections bronchiques et/ou pulmonaires. Egalement il est connu qu'à fortes doses ces produits liquides exposent aux risques indésirables de suintement anal et d'irritation péri-anale.

Les produits gélifiés sont de manipulation plus aisée. Ils sont généralement obtenus soit par mélange de paraffine liquide avec des paraffines semi solides à solides, soit par émulsion d'huile de paraffine liquide avec une phase aqueuse contenant des excipients appropriés. Concernant de telles émulsions, Rector dès 1921 au brevet USP 1389161 décrit le moyen d'obtenir des émulsions transparentes en ajustant l'indice de réfraction de la phase aqueuse à celui de la phase huileuse. W.FF. Whitmore et R.E. Linehan (Ind. and Eng. Chemistry, **21,** p. 878-880, 1929 généralisent cet enseignement par la description de la modification de l'indice de réfraction de l'une ou l'autre phase pour obtenir cette transparence. Ce principe a généré des formules des d'applications diverses, ainsi récemment :
- le brevet européen déposé le 9 septembre 1986 et publié sous le n° 216 557 est relatif à des émulsions translucides de type eau dans huile d'apparence similaire à celle de gelée de paraffine semi solides destinées à l'application sur la peau. Leur phase aqueuse contient un agent humectant, la phase huileuse un agent approprié à la préparation des émulsions eau dans huile et de la paraffine semi solide (F > 38°C) à laquelle il est ajouté éventuellement une huile minérale. Aux exemples illustratifs de l'invention la phase huileuse n'excède pas 70 % et l'agent émulsifiant spécifique aux émulsions eau dans huile est dissout, lors de la préparation, dans la phase huileuse.
- le brevet français déposé le 20 Juillet 1987 et publié sous le n° 2 618 351 a pour objet une émulsion d'huile dans l'eau, gélifiée et transparente, constituée de 50 à 80% en poids d'un liquide huileux hydrophobe, de 0,5 à 5% d'un agent tensio actif hydrosoluble, de 2 à 10% d'un hydrocolloïde, d'agents hydrosolubles et d'eau, étant spécifié que la différence des indices de réfraction de la phase aqueuse et de l'huile n'est pas supérieure à 0,005. Ces gels sont destinés à une utilisation culinaire ou cosmétique, il est précisé que l'hydrocolloïde est la gélatine, et que les compositions, outre l'acide citrique, contiennent comme agent hydrosoluble un sucre ou bien le glycérol ou le sorbitol. Aux exemples illustratifs de l'invention, les émulsions préparées ne contiennent au maximum que 70% en poids de phase huileuse et la phase aqueuse ne contient jamais moins de 16% de gélatine soit 4,8% en poids par rapport à l'émulsion finale. Il est par ailleurs spécifié qu'il est indispensable, pour préparer ces émulsions à haute teneur en phase hydrophobe, d'ajouter l'agent tensioactif hydrosoluble à raison de 0,5 à 5,0% par rapport au gel. L'exemple 3 de cette invention décrit précisément un gel qui, pour 70 parties de vaseline comprend 30 parties d'une solution aqueuse contenant pour 29% d'eau, 45% d'acide citrique, 1 % de lauryl éther sulfate et 25 % de gélatine, soit, pour ce dernier constituant, 7,5 % rapporté au poids de la composition finale ce qui représente un apport protéique important tant au point de vue diététique qu'au point de vue de la consistance du gel qui, vraisemblablement, est de texture quasi solide.

En ce qui concerne les compositions pharmaceutiques laxatives gélifiées proposées et répertoriées au dictionnaire Vidal (déja cité) et quelque soit leur procédé d'obtention, ces produits contiennent du saccharose pour améliorer leur goût, ce qui est signalé être une contre-indication au traitement de la constipation chez les patients diabétiques ou ceux sous régime hypocalorique strict. Pour ce qui est des formes solides proposées, il est commercialisé des produits dans lesquels l'huile liquide est adsorbée sur un support ou bien est présentée sous forme microencapsulée, ces présentations nécessitant des technologies économiquement pénalisantes.

Récemment, à la demande de brevet européen publiée sous le n° 486 353, on propose une composition anhydre caractérisée par l'incorporation de lactulose anhydre dans un mélange d'hydrocarbures paraffiniques purifiés dont le point de fusion se situe entre 45°C et 60°C, les proportions relatives des constituants étant telles que l'activité laxative des hydrocarbures est déclarée modeste au regard de celle du lactulose dont l'activité laxative habituelle est annoncée être exaltée dans la composition revendiquée. Un tel résultat mériterait d'être confirmé car, sans plus de précisions, on comprend que dans l'invention proposée le lactulose anhydre est intimement mélangé à un mélange d'hydrocarbures dont le point de fusion est largement supérieur à la température du corps humain ce qui laisse fortement douter de sa mise à disposition dans le tractus gastro-intestinal et, plus généralement, de l'acceptabilité gustative du produit. Par ailleurs à ce jour on ne peut considérer que le lactulose sous sa forme anhydre soit une solution économiquement et pratiquement satisfaisante compte tenu du coût technologique nécessaire pour obtenir ce produit sous sa forme solide et, dans cet état, de sa propension à se réhydrater spontanément à l'atmosphère ambiante.

De fait, aucune des solutions proposée ou commercialisée pour l'administration a des fins laxatives d'huile de paraffine n'est entièrement satisfaisante, que ce soit pour des raisons d'ordres pratique, économique ou diététique.

La présente invention remédie à cette situation par une composition laxative hypocalorique gélifiée à haute teneur en huile de paraffine liquide associée au lactulose, qui est un disaccharide de synthèse non assimilable ayant lui même des propriétés laxatives reconnues non agressives.

### Sommaire détaillé de l'invention.

Rompant avec l'état de la technique et notamment avec l'enseignement du brevet français n° 2618351, la demanderesse a réalisé de façon surprenante des émulsions associant environ 80% en poids d'huile de paraffine liquide à une phase aqueuse édulcorée contenant du lactulose et gélifiée par un hydrocolloïde, et ce, à l'exclusion remarquable de tout agent tensioactif, afin d'obtenir des compositions gélifiées homogènes, stables, d'aspect translucide et de texture souple non collante.

### Description détaillée de l'invention.

La présente invention a pour objet une nouvelle composition émulsifiée, gélifiée et translucide, caractérisée en ce qu'elle est constituée de 75 à 85 parties en poids d'huile de paraffine liquide et de 25 à 15 parties en poids d'une solution aqueuse édulcorée de lactulose contenant un total de 54 à 66% en poids de matières sèches.

Une composition préférée est celle dans laquelle l'huile de paraffine liquide, dont l'indice de réfraction est compris de 1,473 à 1,483, représente de 76 à 80 parties en poids, pour 20 à 24 parties en poids d'une phase aqueuse dont la teneur en matières sèches est de 57,5 à 62,5%.

La phase aqueuse, outre le lactulose qui en est le constituant majeur, contient également de la gélatine, des adjuvants organo-leptiques, et éventuellement un carbohydrate non assimilable.

Une telle phase est obtenue par le mélange de 90 à 95 parties en poids d'un sirop de lactulose avec 2,5 à 5,5 parties d'une solution aqueuse à 20% de gélatine, 2 à 2,5 parties d'un mélange édulcorant et colorant, et éventuellement un carbohydrate non assimilable, la teneur en matières sèches étant conforme aux spécifications précédemment indiquées et l'indice de réfraction du mélange, déterminé à 20°C, s'ajustant à ±0,010 unité près à celui de l'huile de paraffine .

Plus particulièrement le sirop de lactulose utilisé correspond aux qualités couramment commercialisées, à savoir à des concentrations de 50 à 66% de lactulose. Le sirop titrant 50% est préféré. C'est un sirop limpide, jaune pâle pour lequel à 20°C l'indice de réfraction est compris de 1,430 à 1,490, le pouvoir rotatoire de -36° à -44° et la densité est voisine de 1,310.

La gélatine, conforme aux spécifications de la Pharmacopée européenne (2ème éd.; **330**,1986) est utilisée à raison de 0,5 à 1,1 partie en poids de la phase aqueuse et de préférence de 0,65 à 0,85 parties. Le mélange destiné à édulcorer et colorer la composition comprend les adjuvants organoleptiques nécessaires à la bonne présentation et à la bonne acceptabilité du produit, à savoir, exprimé en poids rapporté au mélange, l'acide citrique pour environ 33%, une composition aromatique qui consiste en un mélange d'arômes naturels et/ou artificiels de framboise et de prune pour environ 60%, un édulcorant de synthèse comme le saccharinate de sodium pour environ 5% et un colorant comme le rouge cochenille pour environ 2%.

Le carbohydrate additif peu ou non assimilable que l'on utilise optionnellement pour ajuster l'indice de réfraction de la phase aqueuse est choisi dans la classe des mono ou des disaccharides reconnus acceptables pour leur utilisation pharmaceutique et leur administration aux sujets diabétiques. Le composé préféré est le sorbitol couramment utilisé comme excipient et édulcorant.

Particulièrement, lorsque la teneur en matières sèches ou l'indice de réfraction de la phase aqueuse ainsi préparée s'avèrent inférieurs ou supérieurs aux normes précédemment définies, on procède à leur ajustement par addition du carbohydrate pour les élever ou, inversement, on dilue par de l'eau pour les diminuer.

Selon l'invention la composition particulièrement préférée répond à la formule centésimale suivante :
- huile de paraffine liquide 78,230 g
- sirop de lactulose à 50% en poids 16,210 g
- sorbitol en poudre 4,200 g
- acide citrique monohydrate 0,150 g
- gélatine 0,160 g
- colorant "rouge cochenille A" 0,012 g
- composition d'arômes artificiels et naturels de framboise et de prune 0,300 g
- saccharine sodique 0,020 g
- eau potable quantité suffisante pour 100,000 g

L'invention vise également un procédé de préparation d'une telle émulsion de type huile dans eau qui consiste à introduire sous agitation appropriée, l'huile de paraffine chauffée à une température de 50 à 80°C dans la phase aqueuse qui, elle-même, est chauffée à une température de 40 à 70°C et qui comprend, la composition d'arômes exceptée, l'ensemble des composés de la phase aqueuse, afin d'obtenir une émulsion gélifiée homogène et translucide à laquelle on ajoute ensuite la composition d'aromatisation et que l'on transfère ensuite dans une cuve de stockage reliée aux machines de remplissage pour répartition de la gelée en pots de verre ou en doses unitaires.

Plus précisement le procédé consiste dans un premier temps à dissoudre le colorant dans la solution de gélatine fraîchement préparée puis à l'ajouter au sirop de lactulose dans lequel on a préalablement solubilisé l'acide citrique, le saccharinate de sodium et éventuellement le sorbitol, puis, dans un second temps, à préparer l'émulsion proprement dite ce qui consiste à introduire l'huile de paraffine préalablement portée à 50/80°C et de préférence à 70°C±5°C, dans la phase aqueuse préparée tel que décrit ci-dessus, chauffée à 40/70°C et de préférence à 55°C±5°C. La débit, et par conséquence la durée d'introduction de l'huile de paraffine, sont tributaires de l'efficacité de l'agitation et de la température à laquelle est réalisée l'opération. Sous agitation de type "turbine" l'introduction peut être réalisée dans un laps de temps compris entre 3 et 45 minutes. Dans les conditions de température préférées indiquées précédemment l'opération peut être réalisée, sous réserve de l'efficacité de l'agitation, en une durée comprise entre 5 et 20 minutes, après quoi on introduit la composition aromatique puis on transfère la venue de produit dans une annexe de stockage avant de le répartir par unités destinées à la commercialisation.

Les exemples et essais décrits dans la partie technique qui suit illustrent, sans pour autant la limiter, l'invention et sa mise en oeuvre.

### Exemple 1 (préféré)

Préparation d'une émulsion gélifiée de composition centésimale :
- huile de paraffine liquide 78,230 g
- sirop de lactulose à 50% en poids 16,210 g
- sorbitol en poudre 4,200 g
- acide citrique monohydrate 0,150 g
- gélatine 0,160 g
- colorant "rouge cochenille A" 0,012 g
- composition d'arômes artificiels et naturels de framboise et de prune 0,300 g
- saccharine sodique 0,020 g
- eau potable quantité suffisante pour 100,000 g

Dans une première étape, dans l'appareil destiné à la préparation de l'émulsion, on introduit 72,945 kg de sirop de lactulose à 50% que l'on a préalablement chauffé à 60°C±5°C et dans lequel on dissout successivement sous agitation modérée 0,675 kg d'acide citrique monohydraté, 0,090 kg de saccharinate de sodium et 18,900 kg de sorbitol en poudre.

Séparément, on dissout dans 3,2 litres d'eau potable 0,054 kg de rouge cochenille A dans lesquels on disperse 0,720 kg de gélatine conforme aux spécifications de la Pharmacopée européenne (2ème ed.; 1986). On abandonne 20 minutes pour gonflement la solution que l'on porte ensuite sur bain-marie à 55°C±5°C avant de l'ajouter dans l'appareil à la solution édulcorée précédemment décrite. L'indice de réfraction du mélange aqueux, mesuré à 20°C est de 1,4740 ±0,010.

Sous agitation on introduit alors en continu et à raison de 50±10 litres par minute 352,035 kg de paraffine liquide préalablement chauffée à 70°C±5°C, en augmentant progressivement la vitesse de l'agitation au fur et à mesure de l'introduction de façon à assurer une incorporation continue de l'huile ajoutée dans l'émulsion gélifiée homogène.

On ajoute ensuite 1,350 kg d'une composition aromatique constituée d'alcoolats d'arômes naturel et artificiel de framboise et d'arôme naturel de prune; on poursuit l'agitation pendant deux minutes puis on transfère l'émulsion dans une cuve de stockage reliée à une machine de conditionnement pour répartition ultérieure en pots de verre ou en godets plastiques pour les doses unitaires.

La gelée ainsi préparée et après refroidissement à 20°C se montre par son aspect, sa consistance et sa transparence similaire à une gelée alimentaire naturelle. Il en est de même par sa manipulation lors de l'administration, elle se tranche facilement et nettement avec une cuillère et, tout en étant suffisamment ferme pour ne pas couler de l'instrument elle présente une texture souple d'un contact gustatif agréable.

Sa stabilité étudiée en pots de verre à 20°C est excellente, après 12 mois l'aspect, le comportement et les caractères organo-leptiques sont parfaitement conservés.

### Exemple 2

Selon le procédé de l'exemple 1, dans l'appareil destiné à la préparation de l'émulsion, on introduit 86,850 kg de sirop de lactulose à 66% en poids, préalablement chauffé à 60°C ±5°C dans lequel on dissout successivement sous agitation modérée 0,675 kg d'acide citrique monohydraté puis 0,090 kg de saccharine sodique.

Séparément on dissout dans 8,2 litres d'eau potable 0,054 kg de rouge cochenille A dans lesquels on disperse 0,720 kg de gélatine. Après gonflement on porte la solution au bain-marie à 55°C±5°C avant de l'ajouter à la solution édulcorée pour ainsi obtenir une phase aqueuse dont la teneur en matières sèches est de 61%.

Sous agitation on introduit alors dans le mélange 352,035 kg de paraffine liquide comme décrit à l'exemple précédent, puis on ajoute 1,350 kg d'une composition aromatique constituée d'alcoolats d'arômes de framboise et de prune. Après deux minutes, l'émulsion gélifiée est transfèrée dans une cuve de stockage avant sa répartition unitaire et son conditionnement.

## Revendications

1. Emulsion gélifiée et translucide, **caractérisée en ce qu'**elle est constituée de 75 à 85 parties en poids d'huile de paraffine liquide et de 25 à 15 parties en poids d'une solution aqueuse édulcorée de lactulose contenant un total de 54 à 66% en poids de matières sèches,

2. Emulsion selon la revendication 1 constituée de 76 à 80 parties en poids d'huile de paraffine liquide d'indice de réfraction compris de 1,473 à 1,483 et de 24 à 20 parties en poids d'une solution aqueuse édulcorée de lactulose contenant un total de 57,5 à 62,5% en poids de matières sèches,

3. Emulsion selon la revendication 1 ou 2 **caractérisée en ce que** la solution aqueuse édulcorée de lactulose est constituée par le mélange de 90 à 95 parties en poids d'un sirop de lactulose avec de 2,5 à 5,5 parties d'une solution aqueuse à 20% de gélatine, 2 à 2,5 parties d'un mélange édulcorant et colorant, et éventuellement un carbohydrate non assimilable, l'indice de réfraction dudit mélange, déterminé à 20°C, s'ajustant à ±0,010 unité près à celui de l'huile de paraffine,

4. Emulsion suivant l'une des revendications précédentes **caractérisée en ce que** le sirop de lactulose utilisé pour la préparation de la solution aqueuse titre de 50 à 66% en poids de produit,

5. Emulsion suivant l'une des revendications précédentes **caractérisée en ce que** la solution aqueuse contient de 0,5 à 1,1 partie en poids de gélatine conforme aux spécifications de la Pharmacopée européenne (2ème éd.; **330**,1986),

6. Emulsion suivant l'une des revendications précédentes **caractérisée en ce que** la solution aqueuse comprend un mélange d'adjuvants organoleptiques composé d'acide citrique, d'une composition aromatique d'arômes naturels et/ou artificiels de framboise et de prune, de saccharinate de sodium, et un colorant comme le rouge cochenille,

7. Emulsion suivant l'une des revendications précédentes **caractérisée en ce que** la solution aqueuse comprend éventuellement du sorbitol à titre de carbohydrate additif pour ajuster l'indice de réfraction de ladite solution,

8. Emulsion dont la composition pondérale est :
- huile de paraffine liquide 78,230 g
- sirop de lactulose à 50% en poids 16,210 g
- sorbitol en poudre 4,200 g
- acide citrique monohydrate 0,150 g
- gélatine 0,160 g
- colorant "rouge cochenille A" 0,012 g
- composition d'arômes artificiels et naturels de framboise et de prune 0,300 g
- saccharine sodique 0,020 g
- eau potable quantité suffisante pour 100,000 g

9. Procédé de préparation d'une émulsion huile dans eau gélifiée homogène et translucide **caractérisé en ce qu'**il consiste à introduire sous agitation dans un laps de temps compris entre 3 et 45 minutes, de 75 à 85 parties en poids l'huile de paraffine dont la température est de 50 à 80°C dans 25 à 15 parties en poids d'une phase aqueuse chauffée à une température de 40 à 70°C constituée par le mélange de 90 à 95 parties en poids d'un sirop de lactulose avec de 2,5 à 5,5 parties d'une solution aqueuse à 20% de gélatine, 2 à 2,5 parties d'un méllange édulcorant et colorant, et éventuellement un carbohydrate non assimilable, l'indice de réfraction dudit mélange, détermine à 20° C, s'ajustant à ±0,010 unité près à celui de l'huile de paraffine, puis à ajouter une composition aromatisante et après transfert à répartir l'émulsion gélifiée et translucide obtenue.

## Patentansprüche

1. Gelierte und transparente Emulsion, **dadurch gekennzeichnet, dass** sie aus 75 bis 85 Gewichtsteilen flüssigem Paraffinöl und 25 bis 15 Gewichtsteilen einer süßstoffhaltigen wässrigen Lactuloselösung mit einem Gesamtgehalt von 54 bis 66 Gew.-% an Trockensubstanz besteht.

2. Emulsion gemäß Anspruch 1, bestehend aus 76 bis 80 Gewichtsteilen flüssigem Paraffinöl mit einem Brechungsindex von 1,473 bis 1,483 und 24 bis 20 Gewichtsteilen einer süßstoffhaltigen wässrigen Lactuloselösung mit einem Gesamtgehalt von 57,5 bis 62,5 Gew.-% an Trockensubstanz.

3. Emulsion gemäß Anspruch 1 oder 2, **dadurch gekennzeichnet, dass** die süßstoffhaltige wässrige Lactuloselösung aus dem Gemisch von 90 bis 95 Gewichtsteilen eines Lactulosesirups mit 2,5 bis 5,5 Teilen einer wässrigen 20%igen Gelatinelösung, 2 bis 2,5 Teilen eines Süßstoff- und Farbstoffgemischs und gegebenenfalls einem nicht assimilierbaren Kohlenhydrat besteht, wobei der Brechungsindex besagten Gemischs, bestimmt bei 20°C, bis auf ±0,010 Einheiten mit dem des Paraffinöls übereinstimmt.

4. Emulsion gemäß einem der vorhergehenden Ansprüche, **dadurch gekennzeichnet, dass** der für die Herstellung der wässrigen Lösung verwendete Lactulosesirup 50 bis 66 Gew.-% Produkt enthält.

5. Emulsion gemäß einem der vorhergehenden Ansprüche, **dadurch gekennzeichnet, dass** die wässrige Lösung 0,5 bis 1,1 Gewichtsteile Gelatine gemäß den Spezifikationen des europäischen amtlichen Arzneibuchs (2. Aufl.; **330**, 1986) enthält.

6. Emulsion gemäß einem der vorhergehenden Ansprüche, **dadurch gekennzeichnet, dass** die wässrige Lösung ein Gemisch organoleptischer Zusätze enthält, das aus Zitronensäure, einer Aromazusammensetzung aus natürlichen und/oder künstlichen Aromen von Himbeere und Pflaume, Saccharinnatrium und einem Farbstoff, wie Cochenillerot, zusammengesetzt ist.

7. Emulsion gemäß einem der vorhergehenden Ansprüche, **dadurch gekennzeichnet, dass** die wässrige Lösung gegebenenfalls Sorbit als zusätzliches Kohlenhydrat enthält, um den Brechungsindex besagter Lösung einzustellen.

8. Emulsion mit der Gewichtszusammensetzung:
- flüssiges Paraffinöl 78,230 g
- Lactulosesirup mit 50 Gew.-% 16,210 g
- Sorbit in Pulverform 4,200 g
- Zitronensäure Monohydrat 0,150 g
- Gelatine 0,160 g
- Farbstoff "Cochenillerot A" 0,012 g
- Zusammensetzung künstlicher und natürlicher Aromen von Himbeere und Pflaume 0,300 g
- Saccharinnatrium 0,020 g
- Trinkwasser in ausreichender Menge für 100,000 g

9. Verfahren zur Herstellung einer gelierten, homogenen und transparenten Öl-in-Wasser-Emulsion, **dadurch gekennzeichnet, dass** es darin besteht, unter Rühren in einem Zeitraum zwischen 3 und 45 Minuten 75 bis 85 Gewichtsteile Paraffinöl, dessen Temperatur 50 bis 80°C beträgt, in 25 bis 15 Gewichtsteile einer wässrigen, auf eine Temperatur von 40 bis 70°C erwärmten Phase, die aus dem Gemisch von 90 bis 95 Gewichtsteilen eines Lactulosesirups mit 2,5 bis 5,5 Teilen einer wässrigen 20%igen Gelatinelösung, 2 bis 2,5 Teilen eines Süßstoff- und Farbstoffgemischs und gegebenenfalls einem nicht assimilierbaren Kohlenhydrat besteht, wobei der Brechungsindex besagten Gemischs, bestimmt bei 20°C, bis auf ±0,010 Einheiten mit dem des Paraffinöls übereinstimmt, einzuführen, dann eine aromatisierende Zusammensetzung zuzugeben und nach Überführung die erhaltene gelierte und transparente Emulsion aufzuteilen.

## Claims

1. Gelled and translucent emulsion, **characterized in that** it consists of 75 to 85 parts by weight of liquid paraffin oil and of 25 to 15 parts by weight of an aqueous sweetened lactulose solution containing a total of 54 to 66% by weight of dry matter.

2. Emulsion according to Claim 1, which consists of 76 to 80 parts by weight of liquid paraffin oil with a refractive index of 1.473 to 1.483, and of 24 to 20 parts by weight of an aqueous sweetened lactulose solution containing a total of 57.5 to 62.5% by weight of dry matter.

3. Emulsion according to Claim 1 or 2, **characterized in that** the aqueous sweetened lactulose solution is made by mixing 90 to 95 parts by weight of a lactulose syrup with 2.5 to 5.5 parts of a 20% aqueous gelatin solution, 2 to 2.5 parts of a sweetening and colouring mixture, and optionally a non-assimilable carbohydrate, the refractive index of the said mixture, determined at 20°C, being adjusted to within ± 0.010 unit to that of the paraffin oil.

4. Emulsion according to one of the preceding claims, **characterized in that** the lactulose syrup used for preparing the aqueous solution has a titre of 50 to 66% by weight of product.

5. Emulsion according to one of the preceding claims, **characterized in that** the aqueous solution contains from 0.5 to 1.1 parts by weight of gelatin in conformity with the specifications of the European Pharmacopoeia (2nd ed.; 330, 1986).

6. Emulsion according to one of the preceding claims, **characterized in that** the aqueous solution comprises a mixture of organoleptic adjuvants composed of citric acid, an aromatic composition of natural and/or artificial raspberry and plum flavours, sodium saccharinate and a colouring such as cochineal red.

7. Emulsion according to one of the preceding claims, **characterized in that** the aqueous solution optionally comprises sorbitol as carbohydrate additive in order to adjust the refractive index of the said solution.

8. Emulsion whose composition by weight is:
- liquid paraffin oil 78.230 g
- lactulose syrup at 50% by weight 16.210 g
- sorbitol powder 4.200 g
- citric acid monohydrate 0.150 g
- gelatin 0.160 g
- "cochineal red A" colouring 0.012 g
- composition of artificial and natural raspberry and plum flavours 0.300 g
- sodium saccharin 0.020 g
- drinking water, sufficient quantity for 100,000 g.

9. Process for preparing a homogeneous and translucent gelled oil-in-water emulsion **characterized in that** it consists in introducing, with stirring, over a time interval of between 3 and 45 minutes, from 75 to 85 parts by weight of the paraffin oil whose temperature is 50 to 80°C into 25 to 15 parts by weight of an aqueous phase heated to a temperature of 40 to 70°C made by mixing 90 to 95 parts by weight of a lactulose syrup with 2.5 to 5.5 parts of a 20% aqueous gelatin solution, 2 to 2.5 parts of a sweetening and colouring mixture, and optionally a non-assimilable carbohydrate, the refractive index of the said mixture, determined at 20°C, being adjusted to within ± 0.010 unit to that of the paraffin oil, and then in adding a flavouring composition and, after transferring, in distributing the gelled and translucent emulsion obtained.
